# EUROPÄISCHE PATENTSCHRIFT

(11) **EP 0 735 033 B1**
(45) Veröffentlichungstag und Bekanntmachung des Hinweises auf die Patenterteilung: **12.07.2000**
(21) Anmeldenummer: 96104467.4
(22) Anmeldetag: 21.03.1996
(51) Int. Cl.: C07D 311/72

(54) **Aminomethylierung von Tocopherolen**
Aminomethylation of tocopherols
Aminométhylation de tocophéroles

(30) Priorität: 28.03.1995 CH 87895
(43) Veröffentlichungstag der Anmeldung: 02.10.1996
(73) Patentinhaber: F. HOFFMANN-LA ROCHE AG, 4070 Basel (CH)
(72) Erfinder: Mueller, Robert Karl, 4058 Basel (CH); Schneider, Heinz, 4104 Oberwil (CH)
(74) Vertreter: Kellenberger, Marcus, Dr.

(56) Entgegenhaltungen:
- EP-A- 0 159 018
- EP-A- 0 319 834
- EP-A- 0 490 815
- US-A- 2 519 863
- ORGANIC REACTIONS, Bd. 1, 1942, Seiten 327-328, XP002009003 BLICKE: "The Mannic Reaction"
- CHEM. PHARM. BULL. (CPBTAL);71; VOL.19 (11); PP.2318-24, EISAI CO., LTD.;EISAI RES. LAB.; TOKYO; JAPAN, XP002009004 NAKAMURA T ET AL: "Tocopherol derivatives. I. Conversion of.beta.-,.gamma.-, and.delta.-tocopherol to.alpha.-tocopherol"
- CHEMICAL ABSTRACTS, Band 73, Nr. , (Columbus, Ohio, US), Seite 383, Spalte 1, Zusammenfassung Nr. 45059z, BACK ET AL.: "Synthesis of ..."

## Beschreibung

Die vorliegende Erfindung betrifft die Aminomethylierung von Tocopherolen, insbesondere die vollständige Aminomethylierung von δ-Tocopherol als solchem oder als Bestandteil eines dieses Tocopherol enthaltenden Gemisches mehrerer sogenannter "Nicht-α-Tocopherole".

Es ist aus der einschlägigen Literatur bekannt, dass die Ueberführung von δ-Tocopherol, das sich von α-Tocopherol durch das Vorhandensein zweier substituierbarer Stellungen (5- und 7-) am aromatischen Ring unterscheidet, in die entsprechenden 5,7-Bis(aminomethyl)-Derivate nur unvollständig gelingt. Eine unvollständige Aminomethylierung von δ-Tocopherol führt nach der zwecks Herstellung von möglichst reinem α-Tocopherol üblicherweise nachgeschalteten katalytischen Reduktion zu einem α/β-Tocopherol-Gemisch, das erst durch einen zusätzlichen Umsetzungs-Zyklus Aminomethylierung + katalytische Reduktion in α-Tocopherol übergeführt werden kann. Als Alternative zu diesem zusätzlichen Zyklus kann man das so erzeugte α-Tocopherol vom Gemisch abtrennen, was selbstverständlich ebenfalls zu einer unzufriedenstellend niedrigen Ausbeute des aus bekannten biologischen Gründen gegenüber den Nicht-α-Tocopherolen bevorzugten α-Tocopherols führt. Aus diesen Gründen haben sich die bisher bekannten Verfahren zur Aminomethylierung von δ-Tocopherol als aufwendig und demzufolge als unwirtschaftlich erwiesen, was auch für die Aminomethylierung von δ-Tocopherol enthaltenden Tocopherolgemischen gilt.

So haben Nakamura und Kijima [Chem. Pharm. Bull. 19(11), 2318-2324 (1971)] δ-Tocopherol mit je acht Moläquivalenten wässriger Dimethylaminlösung und 37%igem Formalin 4 Stunden bei Rückflusstemperatur umgesetzt und dabei ein Gemisch des 5-monosubstituierten Produktes 5-Dimethylaminomethyl-δ-tocopherol (57% Ausbeute) und des 5,7-disubstituierten Produktes 5,7-Bis(dimethylaminomethyl)-δ-tocopherol (nur 31% Ausbeute) erhalten (siehe Seiten 2320 und 2322). Dieses Ergebnis ist offenbar darauf zurückzuführen, dass die 5-Stellung des δ-Tocopherolmoleküls wesentlich reaktiver zur Aminomethylierung ist als dessen 7-Stellung, und dass die Substituierbarkeit der noch freien 7-Stellung durch die Einführung des ersten Aminosubstituenten weiter erschwert wird. Das Problem findet seinen Niederschlag auch in der europäischen Patentpublikation (EP) 159 018 der Henkel Corp., wo die Aminomethylierung eines Tocopherol-gemisches und die anschliessende Trennung der aminomethylierten β-, δ- und γ-Tocopherole von dem nicht umgesetzten α-Tocopherol offenbart sind. Nach katalytischer Hydrierung der aminomethylierten Tocopherole wird das so erhaltene Gemisch von α-, β-, δ- und γ-Tocopherolen nochmals aminomethyliert und hydriert, um ein Produkt mit einem möglichst höheren Gehalt an α-Tocopherol zu erhalten (siehe Seiten 16-19), was wiederum den obenerwähnten "zusätzlichen Umsetzungs-Zyklus" darstellt. Das Problem, das mit der Anwesenheit des δ-Tocopherols verbunden ist, wird unmissverständlich angesprochen, ohne dass eine wirtschaftliche Lösung vorgeschlagen wird. Des weiteren wird bemerkt, dass gemäss dem obigen und weiteren Stand der Technik Aminomethylierungen (Mannich-Reaktionen) in der Regel in Gegenwart eines zugegebenen Lösungsmittels, z.B. eines Alkohols oder eines zyklischen Ethers, z.B. Dioxan, erfolgen.

Aufgabe der vorliegenden Erfindung ist es, die Aminomethylierung von δ-Tocopherol bzw. dieses enthaltenden Tocopherolgemischen möglichst vollständig und demzufolge wirtschaftlich durchzuführen. Das Ziel wird dadurch erreicht, indem man als Mannich-Reagens (Umsetzungsprodukt von Formaldehyd und einem sekundären Amin) eines verwendet, welches separat vor der eigentlichen Aminomethylierung durch Umsetzung von Formaldehyd, vorzugsweise in Form von Paraformaldehyd, mit einem sekundären Amin in vollständiger oder nahezu vollständiger Abwesenheit eines Lösungsmittels hergestellt wurde. Somit betrifft die vorliegende Erfindung das auf diese Weise durchgeführte Aminomethylierungsverfahren.

Das erfindungsgemässe Verfahren zur Aminomethylierung von δ-Tocopherol oder von dieses enthaltenden Tocopherolgemischen ist dadurch gekennzeichnet, dass man als Mannich-Reagens eines verwendet, welches separat vor der eigentlichen Aminomethylierung durch Umsetzung von Formaldehyd mit einem sekundären Amin in vollständiger oder nahezu vollständiger Abwesenheit eines Lösungsmittels hergestellt wurde.

Zur separaten, vor der eigentlichen Aminomethylierung durchzuführen den Herstellung des Mannich-Reagens wird zweckmässigerweise dem sekundären Amin das Formaldehyd, insbesondere in Form von Paraformaldehyd, portionenweise und genügend langsam zugegeben, damit die Temperatur des Reaktionsgemisches nicht zu rasch ansteigt (die Reaktion ist nämlich exotherm). Zudem wird zweckmässigerweise während und nach Beendigung der Zugabe gerührt. Man verwendet pro Moläquivalent des sekundären Amins geeigneterweise zwischen 0,7 und 1,2 Moläquivalenten Formaldehyd, vorzugsweise zwischen 0,9 und 1,1 Moläquivalenten. Da - wie oben erwähnt - die Reaktion exotherm ist, erübrigt sich in der Regel ein Erhitzen des Reaktionsgemisches über die notwendige Anfangsreaktionstemperatur hinaus, die je nach eingesetztem sekundärem Amin normalerweise im Bereich von etwa 50°C bis etwa 70°C liegt. Infolge der exothermen Natur der Reaktion pflegt die Temperatur im Verlauf der Reaktion um ein paar Grad anzusteigen, und zwar normalerweise um bis etwa 20 Grad Celsius, d.h. bis auf etwa 70°C-90°C.

Im Prinzip kann zur Herstellung des Mannich-Reagens jedes beliebige zur Aminomethylierung normalerweise in Frage kommende sekundäre Amin eingesetzt werden. Aus praktischen Gründen verwendet man dazu jedoch insbesondere ein Di(C₁₋₆-alkyl)amin, ein Di(C₂₋₆-hydroxyalkyl)amin, ein Di(C₂₋₆-alkoxyalkyl)amin oder ein cyclisches Amin. Bei dem letztgenannten Amin handelt es sich insbesondere um eine 5- bis 8-gliedrige Ringverbindung, die gegebenenfalls ein weiteres Heteroatom, insbesondere Sauerstoff oder Stickstoff, aufweist und/oder am Ring, auch an einem gegebenenfalls vorhandenen weiteren Stickstoffatom, substituiert sein kann. Im Falle des substituierten cyclischen Amins kommen als Substituenten insbesondere Niederalkylgruppen (vor allem C₁₋₆-Alkylgruppen) in Betracht. Beispiele der Dialkylamine, Di(hydroxyalkyl)amine, Di(alkoxyalkyl)amine und cyclischen Amine sind Dimethylamin, Diethylamin, Di(hydroxyethyl)amin, Di(methoxyethyl)amin, Pyrrolidin, Piperidin, 1-Methyl-piperazin und Morpholin, von denen 1-Methyl-piperazin und Morpholin bevorzugt sind.

Der Verlauf der Reaktion kann auf konventionelle Weise verfolgt werden, z.B. mittels NMR-Analyse eines Musters des Reaktionsgemisches. Dadurch wird festgestellt, dass dieses Gemisch während der Reaktion einen immer grösseren Gehalt am diesbezüglichen Diaminomethan, z.B. Dimorpholinomethan im Falle der Umsetzung von Formaldehyd mit Morpholin als sekundärem Amin, aufweist. Es sind allerdings stets weitere Komponenten vorhanden, und zwar u.a. das entsprechende N-Hydroxymethylamin, z.B. Morpholinomethanol. Normalerweise ist die exotherme Reaktion innert etwa 1 bis 2 Stunden beendet. Das so hergestellte Mannich-Reagens ist bei Raumtemperatur mindestens einige Wochen haltbar.

Dass man zur Herstellung des Mannich-Reagens auf die Verwendung eines Lösungsmittels verzichten kann, ist überraschend und stellt einen Vorteil dieses erfindungsgemässen Verfahrens dar.

Das unter Verwendung des Mannich-Reagens zu erfolgende Aminomethylierungsverfahren wird zweckmässigerweise so durchgeführt, dass man dem δ-Tocopherol bzw. einem dieses enthaltenden Tocopherolgemisch das wie oben beschrieben hergestellte Mannich-Reagens zugibt, oder die umgekehrte Zugabe vornimmt, und das daraus resultierende Reaktionsgemisch unter Rühren im Temperaturbereich zwischen etwa 100°C und etwa 140°C erhitzt. Geeigneterweise verwendet man pro Moläquivalent δ-Tocopherol bzw. der gesamten vorhandenen Nicht-α-Tocopherole (δ-Tocopherol sowie β-Tocopherol und/oder γ-Tocopherol) etwa 2,5 bis 10 Moläquivalente Mannich-Reagens (bezogen auf die zu dessen Herstellung eingesetzte Menge sekundären Amins oder Formaldehyd, je nachdem, welche Menge kleiner und deshalb massgebend ist). Zudem kann die Umsetzung unter Normaldruck oder unter erhöhtem Druck durchgeführt werden, beispielsweise im letzteren Fall indem man in einem verschlossenen Autoklaven reagieren lässt.

Da bekanntlich pflanzliche Oele und Fette, wie beispielsweise Sojaöl, Rapsöl, Baumwollsaatöl, Erdnussöl, Weizenkeimöl, Maisöl, Gerstenöl, Roggenöl, Distelöl und dergleichen, wertvolle natürliche Quellen von Tocopherolen (u.a. α- und δ-Tocopherol) sind, können solche Oele oder deren Distillate, die einen höheren Gehalt an Tocopherolen aufweisen und weniger unerwünschte andere Bestandteile, z.B. Sterole, freie und veresterte Fettsäuren, Wachse und Glyzeride, enthalten, als Edukt im erfindungsgemässen Aminomethylierungsverfahren Verwendung finden. Insbesondere Distelöl und Sojaöl erweisen sich als wertvolle Quellen von Tocopherolen, u.a. α-Tocopherol und des in dieses erfindungsgemäss überzuführenden δ-Tocopherols.Es spielt natürlich keine Rolle, dass sich u.a. α-Tocopherol selber im Edukt befindet, da das α-Tocopherol die Ueberführung des δ-Tocopherols in α-Tocopherol nicht verhindert und selber unreagiert im Verfahrensprodukt bleibt.

Der Verlauf der Aminomethylierung wird zweckmässigerweise mittels gaschromatographischer(GC-) Analyse des Reaktionsgemisches verfolgt, und zwar vorteilhafterweise mittels GC-Analyse silylierter Proben. Dabei werden die abnehmenden Gehalte am diesbezüglichen 5-Aminomethyl-δ-Tocopherol und die entsprechend zunehmenden Gehalte am diesbezüglichen 5,7-Bis(aminomethyl)- δ-Tocopherol festgestellt. Normalerweise ist die (nahezu) vollständige Aminomethylierung innert etwa 3 bis 8 Stunden beendet. Danach wird aufgearbeitet, und zwar zweckmässigerweise indem man das Gemisch abkühlt und das überschüssige Mannich-Reagens in der Regel destillativ abtrennt.

Auch bei der soeben beschriebenen Aufarbeitung liegt ein weiterer Vorteil des erfindungsgemässen Aminomethylierungsverfahrens vor: das destillativ abgetrennte überschüssige Mannich-Reagens kann nach Regenerierung zu einer weiteren Aminomethylierung verwendet werden, und zwar mit ebensogutem Erfolg. Die Rezyklisierung (Wiederverwendung) des Mannich-Reagens kann sogar mit gutem Erfolg mehrmals wiederholt werden. Die Regenerierung selber wird zweckmässigerweise so durchgeführt, dass man dem destillativ abgetrennten überschüssigen Mannich-Reagens Wasser bei Raumtemperatur und unter Rühren zugibt, das resultierende wässrige Gemisch auf etwa 80°C erhitzt, und anschliessend Formaldehyd, vorzugsweise in Form von Paraformaldehyd, unter Rühren zugibt. Dann wird vorteilhaft weiter bei dieser Temperatur, beispielsweise weitere etwa 3 Stunden, gerührt. Geeigneterweise verwendet man etwa äquimolare Mengen der drei Reaktionsteilnehmer in dieser Regenerierung, wobei die Abweichung vorzugsweise nicht mehr als etwa 10% beträgt.

Als weitere Alternative zur Verwendung des im wesentlichen lösungsmittelfreien hergestellten Mannich-Reagens im Aminomethylierungsverfahren kann man in diesem Verfahren ein Mannich-Reagens einsetzen, welches separat vor der eigentlichen Aminomethylierung durch Umsetzung von einem aus einem sekundären Amin hergestellten Diaminomethan, z.B. Dimorpholinomethan, mit Formaldehyd (vorzugsweise als Paraformaldehyd) sowie mit Wasser in etwa äquimolaren Mengen hergestellt wurde. Dieses Verfahren bildet einen weiteren Aspekt der vorliegenden Erfindung. Zweckmässigerweise beträgt eine Abweichung dieser äquimolaren Mengen nicht mehr als etwa 10%. Die drei Reaktionsteilnehmer werden geeigneterweise auf etwa 70-90°C erhitzt und bei dieser Temperatur in der Regel nicht mehr als etwa drei Stunden, vorzugsweise etwa 1-1 1/2 Stunden, miteinander reagieren gelassen. Im Gegensatz zur Herstellung des Mannich-Reagens aus dem sekundären Amin und Formaldehyd ist die vorliegende Reaktion nicht (besonders) exotherm. Das Produkt unterscheidet sich in seiner Zusammensetzung und Wirkung kaum vom Produkt (Mannich-Reagens) des im wesentlichen lösungsmittelfreien Verfahrens und vom regenerierten Mannich-Reagens.

Das Verfahren zur ofen beschriebenen Regenerierung des destillativ abgetrennten überschüssigen Mannich-Reagens bildet einen noch weiteren Aspekt der vorliegenden Erfindung, wie auch die Verwendung des so regenerierten Mannich-Reagenzien in Verfahren zur Aminomethylierung von δ-Tocopherol oder von dieses enthaltenden Tocopherolgemischen.

Die obenerwähnten Diaminomethane sind entweder bekannt oder können nach an sich bekannten Methoden, insbesondere ausgehend von den entsprechenden sekundären Aminen, hergestellt werden.

Um schliesslich zum erwünschten α-Tocopherol zu gelangen, kann das Produkt der erfindungsgemässen Aminomethylierung, welches einen hohen Anteil am diaminomethylierten δ-Tocopherol und je nach eingesetztem Tocopherolgemisch (Edukt) monoaminomethyliertes β- und/oder γ-Tocopherol sowie unverändertes, ursprünglich vorhandenes α-Tocopherol enthält, reduziert, z.B. katalytisch hydriert, werden, was auf an sich bekannter Weise [siehe beispielsweise EP 159 018, USP 2,486,539 sowie USP 2,519,863] erfolgen kann. Zu diesem Zweck wird die Hydrierung vorzugsweise mit einem Palladium-Katalysator in einem nichtpolaren Lösungsmittel, z.B. einem Dialkylether, insbesondere tert.Butylmethylether, oder einem Kohlenwasserstoff, z.B. n-Hexan oder Cyclohexan, durchgeführt. Die Hydrierung benötigt erfahrungsgemäss Reaktionstemperaturen von etwa 150°C bis etwa 210°C, einen Wasserstoffdruck von etwa 15 bis etwa 50 bar sowie eine Reaktionszeit von etwa 2 bis etwa 10 Stunden. Die Isolierung und Reinigung des erwünschten α-Tocopherols können ebenfalls nach an sich bekannten Methoden erfolgen.

Viele erfindungsgemäss aminomethylierte δ-Tocopherole sind neu. Diese neuen Bis(aminomethyl)-δ-tocopherole, d.h. die aus den diesbezüglichen Mannich-Reagenzien und δ-Tocopherol erfindungsgemäss herstellbaren disubstituierten [Bis(aminomethylierten)] δ-Tocopherole, haben die allgemeine Formel 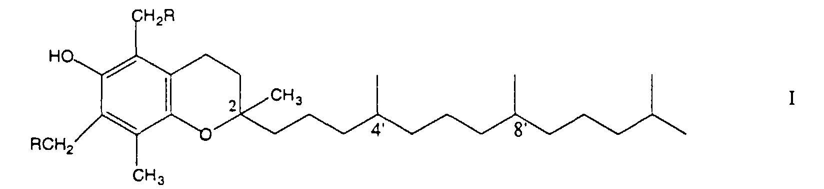 worin R eine Di(C₂₋₆-alkyl)aminogruppe, eine Di(C₂₋₆-hydroxyalkyl)aminogruppe, eine Di(C₂₋₆-alkoxyalkyl)aminogruppe oder eine 5- bis 8-gliedrige cyclische Aminogruppe, die gegebenenfalls ein weiteres Heteroatom, insbesondere Sauerstoff oder Stickstoff, aufweist und/oder am Ring, auch an einem gegebenenfalls vorhandenen weiteren Stickstoffatom, substituiert sein kann, bedeutet.

Im Fall der substituierten cyclischen Aminogruppe kommen als Substituenten insbesondere Niederalkylgruppen (vor allem C₁₋₆-Alkylgruppen) in Betracht. Beispiele der Di(C₂₋₆-alkyl)amino-, Di(C₂₋₆-hydroxyalkyl)amino-, Di(C₂₋₆-alkoxyalkyl)amino- und 5- bis 8-gliedrigen cyclischen Aminogruppen (R) sind Diethylamino, Di(hydroxyethyl)amino, Di(methoxyethyl)amino, Pyrrolidino, Piperidino, N-Methylpiperazino und Morpholino.

Die Formel I, bei der die in der Tocopherol-Chemie übliche (mit einfachen Strichen) abgekürzte Darstellungsweise benutzt wird, umfasst isomere Formen, insbesondere optisch aktive Isomere, sowie Gemische hiervon, sofern nicht ausdrücklich anders erwähnt. Als Beispiele der chiralen (optisch aktiven) Zentren seien erwähnt das die Methylgruppe und die 4,8,12-Trimethyltridecylgruppe tragende Kohlenstoffatom (in der Formel I als 2 bezeichnet) sowie die 4- und 8-Kohlenstoffatome besagter Trimethyltridecylgruppe (als 4' bzw. 8' bezeichnet).

Sechs obenerwähnte spezifische neue sowie drei weitere neue Verbindungen der Formel I wurden nach Chromatographie an Kieselgel als farblose oder leicht gelbliche, viskose Oele erhalten, und zwar ausgehend von natürlichem δ-Tocopherol jeweils mit der 2R,4'R,8'R-Konfiguration. Die erhaltenen ¹H-NMR-Spektren dieser disubstituierten δ-Tocopherole weisen keine Signale zwischen 5 und 9 ppm auf, was die vollständige Substitution des Benzolkerns nachweist. In der nachfolgenden Tabelle sind die physikalischen Daten der neun Verbindungen angegeben, wobei "c%" Konzentration in Gewichtsprozent bedeutet:

| R | [α]^{D} (bei 20°C, c = 1%) | Gehalt der analysierten Probe (GC Fl.-%) | Auszug ¹H-NMR-Spektrum (CDCl₃): R-CH₂- | Massenspektrum |
|---|---|---|---|---|
| N(Ethyl)₂ | + 1,3° in C₂H₅OH | 98,7% | 3,67 (s,2H); 3,59 (s,2H). | EI-MS: 572 (6, M); 499 (57, M -C₄H₁₁N); 470 (80); 428 (100, 499 -C₄H₉N); 426 (57, 499 -C₄H₁₁N). |
| N(n-Propyl)₂ | + 1,4°, in CHCl₃ | 98,6% | 3,66 (s, 2H); 3,57 (s, 2H). | ISP-MS: 629,7 (100, M+H); 528,4 (39, M -C₆H₁₄N). |
| N(n-Butyl)₂ | + 2,7°, in CHCl₃ | 98,0% | 3,65 (s, 2H); 3,56 (s, 2H). | ISP-MS: 685 (100, M+H); 556 (15, M -C₈H₁₈N). |
| N(n-Butyl)(methyl) | + 1,2°, in CHCl₃ | 99,4% | 3,58 (s, 2H); 3,51 (s, 2H). | EI-MS: 601 (7, M+H); 543 (7, M -C₄H₉); 513 (100, M -C₅H₁₃N); 498 (14, M -C₅H₁₃N -CH₃); 456 (65, M -C₅H₁₃N -C₄H₉); 428 (85, 513 -C₅H₁₁N); 165 (77, C₁₀H₁₃O₂). |
| N(Methoxyethyl)₂ | + 2,3°, in CHCl₃ | 99,1% | 3,78 (s, 2H); 3,71 (s, 2H). | EI-MS: 693 (5, M+H); 560 (21, M -C₆H₁₄NO₂); 500 (19, M -C₆H₁₅NO₂ -C₃H₇O); 428 (12, 560 -C₆H₁₄NO₂); 426 (13, M -2 C₆H₁₅NO₂); 165 (95, C₁₀H₁₃O₂); 133 (18, C₆H₁₅NO₂); 88 (100, C₄H₁₀NO). |
| Morpholino | + 2,1°, in C₂H₅OH | 96,2% | 3,63 (s,2H); 3,56 (s,2H). | EI-MS: 600 (10, M); 513 (100, M -C₄H₉NO); 428 (85, 513-C₄H₇NO); 426 (90, 513 -C₄H₉NO). |
| Piperidino | + 2,3°, in C₂H₅OH | 94,6% | 3,57 (s,2H); 3,50 (s,2H). | ISP-MS: 597 (20, [M+H]⁺); 512 (100, 597 -C₅H₁₁N). |
| Pyrrolidino | + 2,4°, in C₂H₅OH | 92,2% | 3,76 (s,2H); 3,71 (s,2H). | EI-MS: 568 (16, M); 497 (85, M -C₄H₉N); 428 (100, 497 -C₄H₇N); 427 (60, 497 -C₄H₈N). |
| N-Methylpiperazino | + 2,8°, in C₂H₅OH | 99,3% | 3,63 (s,2H); 3,56 (s,2H). | EI-MS: 626 (2, M); 526 (34, M -C₅H₁₂N₂); 428 (12, 526 -C₅H₁₀N₂); 426 (11, 526 -C₅H₁₂N₂); 99 (100, -C₅H₁₁N₂⁺). |

Besonders bevorzugte Bis(aminomethyl)-δ-tocopherole der Formel I sind das 5,7-Bis(morpholinomethyl)-δ-tocopherol und das 5,7-Bis(N-methylpiperazinomethyl)-δ-tocopherol.

Bei dem Aminomethylierungsverfahren, das zur Herstellung der disubstituierten δ-Tocopherole dient, besteht ein weiterer Vorteil: falls das Edukt (δ-Tocopherol) isomerenrein, z.B. der 2R,4'R,8'R-Konfiguration, ist, bleibt die stereochemische Reinheit im 5,7-disubstituierten (aminomethylierten) Produkt erhalten.

Die vorliegende Erfindung wird durch die nachfolgenden Beispiele veranschaulicht:

### Beispiel 1

### Herstellung eines "Mannich-Reagens"

Zu 87,0 ml (87,0 g, 1,0 Mol) Morpholin werden bei etwa 70°C und unter Rühren 30,0 g (1,0 Mol) Paraformaldehyd portionenweise so zugegeben, dass die Temperatur auf maximal 80°C ansteigt, wobei die Zugabe insgesamt etwa 30 Minuten dauert. Nach etwa 1 1/2 Stunden ist die exotherme Reaktion unter Bildung einer farblosen Flüssigkeit beendet. Gemäss ¹H-NMR- und ¹³C-NMR-Analyse liegt ein Mehrkomponentengemisch vor, das etwa zur Hälfte aus Dimorpholinomethan besteht. Dieses Gemisch (das "Mannich-Reagens") bleibt bei Raumtemperatur mindestens einige Wochen haltbar.

### Beispiel 2

### Herstellung eines "Mannich-Reagens"

Zu 1,0 Mol sekundärem Amin [jeweils Diethylamin, Di(n-propyl)amin, Di(n-butyl)amin, Methylbutylamin, Di(methoxyethyl)amin, Morpholin, 1-Methyl-piperazin, Piperidin oder Pyrrolidin] gibt man bei 70-80°C (im Falle von Diethylamin allerdings bei 50°C) und unter Rühren 30,0 g (1,0 Mol) Paraformaldehyd portionenweise so zu, dass die Temperatur auf maximal 90°C ansteigt. Nach beendeter Zugabe, die je nach sekundärem Amin etwa 15 bis 45 Minuten in Anspruch nimmt, rührt man noch 2 Stunden bei 70-80°C und kühlt auf Raumtemperatur ab. Die verbleibenden farblosen bis leicht bräunlichen, in einigen Fällen aus 2 Phasen bestehenden Flüssigkeiten ("Mannich-Reagenzien") können direkt für die Aminomethylierung von Nicht-α-Tocopherolen oder Tocopherolgemischen eingesetzt werden.

### Beispiel 3

### Herstellung eines "Mannich-Reagens" (Alternative)

Zu 102,2 g (1,0 Mol) Bis(dimethylamino)methan tropft man unter Rühren innerhalb von 10 Minuten 18,0 g (1,0 Mol) Wasser, wobei eine leichte Erwärmung von 23 auf 30°C eintritt. Dann erhitzt man das resultierende zweiphasige Gemisch auf 80°C, trägt innerhalb von etwa 10 Minuten portionsweise 30,0 g (1,0 Mol) Paraformaldehyd ein, rührt noch 2 Stunden bei 80°C und lässt auf Raumtemperatur abkühlen. Die erhaltene farblose, klare Lösung ("Mannich-Reagens") kann direkt für die Aminomethylierung von Nicht-α-Tocopherolen oder Tocopherolgemischen eingesetzt werden.

### Beispiel 4

### Aminomethylierung mit einem Mannich-Reagens und anschliessende katalytische Hydrierung

Zu einem hauptsächlich aus δ-Tocopherol bestehenden Tocopherol-Gemisch (42 g, etwa 0,1 Mol: 85,5% δ-, 4,6% γ- sowie 0,5% α-Tocopherol) werden unter Rühren 46,8 g (0,4 Mol) Mannich-Reagens (gemäss Beispiel 1 hergestellt) zugegeben. Man erhitzt das homogene Gemisch 9 Stunden bei 130°C, währenddessen entstehendes Wasser abgedampft wird. Nach dieser Reaktionszeit besteht das Aminomethylierungsprodukt gemäss gaschromatographischer (GC-) Analyse aus 5,7-Bis(morpholinomethyl)-δ-tocopherol (92,5%), 5-Morpholinomethyl-γ-tocopherol (4,2%), 5-Morpholinomethyl-δ-tocopherol (0,6%) sowie α-Tocopherol (0,6%).

Das obige Aminomethylierungsprodukt wird unter Verwendung von 10%igem Palladium auf Kohlenstoff als Katalysator in tert.Butylmethyläther (etwa 10%ige Lösung) bei 180°C und 20 bar 20 Stunden hydriert. Auf diese Weise erhält man eine fast quantitative Ausbeute von α-Tocopherol (GC-Analyse: 97,0% α-, 1,9% β- sowie 0,9% γ-Tocopherol).

### Beispiel 5

### Aminomethylierung mit einem Mannich-Reagens

440 g (1,0 Mol) eines aus 4,6% α-, 1,0% β-, 58,8% γ- sowie 30,2% δ-Tocopherol bestehenden Tocopherol-Gemisches werden unter Rühren in 705 g eines gemäss Beispiel 1 hergestellten Mannich-Reagens gelöst, wobei die Temperatur des Reaktionsgemisches auf etwa 35°C ansteigt (wohl auf beginnende Aminomethylierung zurückzuführen). Nach etwa einer Stunde wird auf 110°C Innentemperatur aufgeheizt (Heizbadtemperatur etwa 140°C), währenddessen entstehendes Wasser abgedampft wird. Die Reaktionstemperatur steigt innert weiterer 1 1/2 Stunden auf 130°C an, und nach weiteren 4 Stunden bei dieser Temperatur ist die Reaktion beendet. Das Aminomethylierungsprodukt (1005 g) besteht gemäss GC-Analyse aus α-Tocopherol (4,0%), 7-Morpholinomethyl-β-tocopherol (2,0%), 5-Morpholinomethyl-γ-tocopherol (59,8%), 5,7-Bis(morpholinomethyl)-δ-tocopherol (32,6%) sowie 5-Morpholinomethyl-δ-tocopherol (0,4%).

Anschliessend wird das Aminomethylierungsprodukt in einem Fallfilmverdampfer vom überschüssigen Mannich-Reagens (hauptsächlich Dimorpholinomethan) befreit.

### Beispiel 6

### Aminomethylierung mit einem Mannich-Reagens und anschliessende katalytische Hydrierung

218,35 g (0,5 Mol) eines aus 4,6% α-, 1,0% β-, 58,8% γ- sowie 30,2% δ-Tocopherol bestehenden Tocopherol-Gemisches werden unter Rühren in 300 g (2,56 Mol) eines gemäss Beispiel 1 hergestellten Mannich-Reagens gelöst. Bei einer Heizbadtemperatur von 120°C wird nach etwa 30 Minuten eine Reaktionstemperatur von 105°C erreicht, wobei entstehendes Wasser abgedampft wird. Innert weiterer 4 Stunden steigt die Reaktionstemperatur auf 117°C an. Man rührt das Reaktionsgemisch weitere 4 3/4 Stunden bei dieser Temperatur und lässt es anschliessend auf Raumtemperatur abkühlen. Das Aminomethylierungsprodukt (456,4 g) besteht gemäss GC-Analyse aus α-Tocopherol (3,55%), 7-Morpholinomethyl-β-tocopherol (1,7%), 5-Morpholinomethyl-γ-tocopherol (60,1%), 5,7-Bis(morpholinomethyl)-δ-tocopherol (32,86%) sowie 5-Morpholinomethyl-δ-tocopherol (0,2%).

Anschliessend wird das Aminomethylierungsprodukt in einem Fallfilmverdampfer vom überschüssigen Mannich-Reagens (hauptsächlich Dimorpholinomethan) befreit.

Das obige, von überschüssigem Mannich-Reagens befreite Aminomethylierungsprodukt wird unter Verwendung von 10%igem Palladium auf Kohlenstoff als Katalysator in tert.Butylmethyläther (etwa 10%ige Lösung) bei 180°C und 30 bar 5 Stunden hydriert. Auf diese Weise erhält man eine fast quantitative Ausbeute von α-Tocopherol (GC-Analyse: 98,0% α-, 0,8% β- sowie 1,2% γ-Tocopherol).

### Beispiel 7

### Aminomethylierung mit einem Mannich-Reagens im Autoklaven

172,44 g (0,4 Mol) eines aus 0,4% α-, 64,9% γ- sowie 30,3% δ-Tocopherol bestehenden Tocopherol-Gemisches werden unter Rühren in 247,5 g eines gemäss Beispiel 1 hergestellten Mannich-Reagens gelöst. Das Reaktionsgemisch wird in einen 2 l-Laborrührautoklaven (Büchi BEP 280 Typ IV, Stahl) eingefüllt. Der Autoklav wird verschlossen und das Reaktionsgemisch unter Rühren auf 118°C (Innentemperatur) erhitzt. Der vollständige Verlauf der Umsetzung wird durch gaschromatographische Bestimmung der abnehmenden Gehalte an 5-Morpholinomethyl-δ-tocopherol verfolgt: Reaktionszeit 2 Stunden: 1,2%; Reaktionszeit 3 Stunden: 0,3%; Reaktionszeit 4 Stunden: < 0,1%. Nach insgesamt 4 1/2 Stunden (Druckanstieg auf 4,1 bar) wird das Reaktionsgemisch auf 30°C abgekühlt. Das so erhaltene Aminomethylierungsprodukt besteht gemäss GC-Analyse aus α-Tocopherol (0,35%), 5-Morpholinomethyl-y-tocopherol (66,3%) sowie 5,7-Bis(morpholinomethyl)-δ-tocopherol (33,4%).

### Beispiel 8

### Herstellung eines Mannich-Reagens und anschliessende Aminomethylierung

6,7 ml (6 g, 60 mMol) 1-Methylpiperazin werden unter Argon in einem mit Innenthermometer und Magnetrührer versehenen 50 ml-Dreihalskolben bei Raumtemperatur vorgelegt und auf 60°C erwärmt. Innert 10 Minuten werden portionenweise 1,8 g (60 mMol) Paraformaldehyd zum 1-Methylpiperazin zugegeben. Anschliessend wird das Gemisch weitere 30 Minuten bei 60°C gerührt.

Zu der so erhaltenen homogenen Lösung (dem Mannich-Reagens) werden anschliessend 4,2 g (etwa 10 mMol) δ-Tocopherol zugegeben, und das Reaktionsgemisch innerhalb von 30 Minuten auf 120°C und weitere etwa 2 1/2 Stunden bei dieser Temperatur erhitzt. Man verfolgt den Reaktionsverlauf mittels GC-Analyse von silylierten Proben, um die prozentuellen Gehalte am mono- und disubstituierten Produkt [hauptsächlich 5-(N-Methyl-piperazinomethyl)-δ-tocopherol bzw. 5,7-Bis(N-methyl-piperazinomethyl)-δ-tocopherol] zu bestimmen, und zwar mit den nachfolgenden Ergebnissen:

| Reaktionszeit (Stunden ab anfänglicher Zugabe) | Gehalt an | |
|---|---|---|
| | monosubst. Produkt | disubst. Produkt |
| 0,25 | 5,9% | 84,6% |
| 1,5 | 1,3% | 92,8% |
| 3,0 | 0% | 93,6% |

### Beispiel 9

### Aminomethylierung von δ-Tocopherol mit einem Mannich-Reagens im Autoklaven und anschliessende katalytische Hydrierung

In einem 185 ml-Stahlautoklaven mit mechanischem Rührer gibt man zu 22,32 g d-δ-Tocopherol [der Firma Sigma; etwa 90%ig, Gehalt an: δ-Tocopherol 86,6%, 48,0 mmol; γ-Tocopherol 3,7%, 1,96 mmol; α-Tocopherol 0,1%, 0,04 mmol; Gesamt-Tocopherole 90,4%, 50,0 mmol; bestimmt mittels GC der Acetate, interner Standard Squalan; stereochemische Reinheit: 94,95% R,R,R-δ- und 4,87% R,R,R-γ-Homologen, gesamt 99,82% R,R,R-Tocopherole; bestimmt mittels HPLC des Methylether-Derivates an Chiracel OD, einer käuflichen Chromatographie-Säule der Firma Daicel] gemäss den Angaben in der nachfolgenden Tabelle 0,3 bzw. 0,5 Mol (6 bzw. 10 Mol-Aequivalente bezogen auf Gesamt-Tocopherole) Mannich-Reagens (gemäss Beispiel 1, 2, 3, 8 oder 11 hergestellt) sowie in zwei Beispielen als Lösungsvermittler N,N,N',N'-Tetramethylethylendiamin (100 Gew.% bezogen auf eingesetztes Mannich-Reagens) und rührt im verschlossenen Autoklaven bei 600 U/ Min. während 24 Stunden bei 120 bzw. 140°C. Es fallen zumeist braune Oele an. In einigen Fällen scheidet sich eine kleine (maximal 13 Gew.%) untere Phase ab, die abgetrennt und verworfen wird. Zur Analyse silyliert man eine kleine Probe des Rohproduktes [N,O-Bis(trimethylsilyl)trifluoracetamid/ Pyridin] und erhält die in der nachfolgenden Tabelle angegebenen Ergebnisse.

| Amin-Komponente NR¹R² | Reaktionstemperatur (°C) | Mol-Aequivalente Mannich-Reagens | zugesetzter Lösungsvermittler | Verhältnis (GC FI. % nach Silylierung) 1) | |
|---|---|---|---|---|---|
| | | | | monosubstituiertes Produkt | disubstituiertes Produkt |
| R¹ = R² = Methyl | 140 | 10 | - | 0,9 | 94,4 |
| R¹ = R² = Ethyl | 140 | 10 | N,N,N',N'-Tetramethylethylendiamin | 3,7 | 92,7 |
| R¹ = R² = n-Propyl | 140 | 6 | N,N,N',N'-Tetramethylethylendiamin | 1,1 | 95,5 |
| R¹ = R² = n-Butyl | 140 | 10 | - | 2,5 | 93,5 |
| R¹ = Methyl, R² = n-Butyl | 120 | 6 | - | 2,0 | 94,6 |
| R¹ = R² = Methoxyethyl | 140 | 10 | - | 2,5 | 93,8 |
| Morpholino | 120 | 6 | - | 0 | 94,4 |
| N-Methylpiperazino | 120 | 6 | - | 0 | 95,6 |
| Pyrrolidino | 120 | 6 | - | 3,3 | 92,4 |
| Piperidino | 140 | 10 | - | 1,2 | 95,3 |

| | | | | | |
|---|---|---|---|---|---|
| 1) monosubstituiertes Produkt = 5-Dialkylaminomethyl-δ-Tocopherol, disubstituiertes Produkt = 5,7-Bis(dialkylaminomethyl)-δ-Tocopherol; daneben zusätzlich 3,4-4,4% 5-Dialkylaminomethyl-γ-Tocopherol und Spuren (etwa 0,1%) α-Tocopherol. | | | | | |

Die so erhaltenen rohen Aminomethylierungsprodukte werden in Ansätzen von 1-10 mmol mit 10% Palladium auf Kohlenstoff (Degussa E 101 N/D, 1,0 g für 10 mmol) in tert.Butylmethylether als Lösungsmittel während 20 Stunden und Rühren bei 180°C/ 20 bar Wasserstoffdruck hydriert. Nach beendeter Reaktion wird über Speedex (Filterhilfsmittel) vom Katalysator abfiltriert, mit tert.Butylmethylether nachgewaschen, das Lösungsmittel unter vermindertem Druck abdestilliert und die Ausbeute an Tocopherolen mittels GC (nach Acetylierung mit Acetanhydrid/ Pyridin/Dimethylaminopyridin) quantitativ bestimmt. In der nachfolgenden Tabelle sind einige Details angegeben.

| Eingesetztes Rohprodukt : Aminomethylierungsprodukt; Amin-Komponente NR¹R² | Konzentration der Hydrierlösung (g eingesetztes Rohprodukt pro 100 ml) | Chemische Ausbeute an Tocopherolen (GC der Acetate, interner Standard Squalan) in % (bezogen auf das gesamte eingesetzte Tocopherol) | | |
|---|---|---|---|---|
| | | alpha-Tocopherol | beta-Tocopherol | gamma-Tocopherol |
| R¹ = R² = n-Propyl | 6 | 94,4 | 1,9 | 1,5 |
| R¹ = R² = n-Butyl | 25 | 90,0 | 2,6 | 1,0 |
| R¹ = Methyl, R² = n-Butyl | 13 | 96,5 | 2,9 | 0,0 |
| R¹ = R² = Methoxyethyl | 24 | 81,1 | 2,3 | 0,9 |
| Morpholino | 13 | 90,3 | 1,2 | 0,6 |
| N-Methylpiperazino | 16 | 87,5 | 4,3 | 1,6 |
| Pyrrolidino | 2 | 82,2 | 4,9 | 2,3 |
| Piperidino | 2 | 83,4 | 1,4 | 0,9 |

### Beispiel 10

### Regenerierung eines Mannich-Reagens

Der Ueberschuss am zur Aminomethylierung verwendeten Mannich-Reagens kann nach beendeter Aminomethylierung durch Destillation zurückgewonnen werden. Ein solches Destillat [205,8 g wasserklare Flüssigkeit; gemäss ¹H-NMR bestehend aus einem etwa 6:1-Gemisch von Dimorpholinomethan (etwa 1,0 Mol) und N-Hydroxymethyl-morpholin] wird bei Raumtemperatur unter Rühren mit 18 g (1,0 Mol) Wasser versetzt. Man erhitzt das resultierende wässrige Gemisch im Oelbad auf 80°C und gibt innert einer Stunde portionenweise 30 g (1,0 Mol) Paraformaldehyd zu. Es wird weitere 3 Stunden gerührt, wobei eine farblose, fast klare Lösung entsteht. Das ¹H-NMR-Spektrum dieses so regenerierten Mannich-Reagens entspricht praktisch genau dem ¹H-NMR-Spektrum eines frisch hergestellten Mannich-Reagens (wie beispielsweise gemäss Beispiel 1 hergestellt).

## Patentansprüche

1. Verfahren zur Aminomethylierung von δ-Tocopherol oder von dieses enthaltenden Tocopherolgemischen, dadurch gekennzeichnet, dass man als Mannich-Reagens eines verwendet, welches separat vor der eigentlichen Aminomethylierung durch Umsetzung von Formaldehyd, insbesondere Paraformaldehyd, mit einem sekundären Amin in vollständiger oder nahezu vollständiger Abwesenheit eines Lösungsmittels hergestellt wurde.

2. Verfahren nach Anspruch 1, worin man pro Moläquivalent des sekundären Amins zwischen 0,7 und 1,2 Moläquivalenten Formaldehyd verwendet.

3. Verfahren nach Anspruch 1 oder 2, worin man die Temperatur des aus Formaldehyd und dem sekundären Amin bestehenden Reaktionsgemisches im Bereich von etwa 70°C bis etwa 90°C hält.

4. Verfahren zur Aminomethylierung von δ-Tocopherol oder von dieses enthaltenden Tocopherolgemischen, dadurch gekennzeichnet, dass man als Mannich-Reagens eines verwendet, welches separat vor der eigentlichen Aminomethylierung durch Umsetzung von einem aus einem sekundären Amin hergestellten Diaminomethan mit Formaldehyd, insbesondere Paraformaldehyd, sowie mit Wasser in etwa äquimolaren Mengen hergestellt wurde.

5. Verfahren nach einem der Ansprüche 1 bis 4, worin als sekundäres Amin ein Di(C₁₋₆-alkyl)amin, ein Di(C₁₋₆-hydroxyalkyl)amin, ein Di(C₂₋₆-alkoxyalkyl)amin oder ein cyclisches Amin, das eine 5- bis 8-gliedrige Ringverbindung ist und gegebenenfalls ein weiteres Heteroatom, insbesondere Sauerstoff oder Stickstoff, aufweist und/oder am Ring, auch an einem gegebenenfalls vorhandenen weiteren Stickstoffatom, substituiert sein kann, verwendet wird.

6. Verfahren nach Anspruch 5, worin das sekundäre Amin ein Di-(C₁₋₆-alkyl)amin oder ein cyclisches Amin ist.

7. Verfahren nach Anspruch 6, worin das sekundäre Amin 1-Methyl-piperazin oder Morpholin ist.

8. Verfahren nach einem der Ansprüche 1-7, worin man das aus dem Mannich-Reagens und dem δ-Tocopherol bzw. dieses enthaltenden Tocopherolgemisch bestehende Reaktionsgemisch unter Rühren im Temperaturbereich zwischen etwa 100°C und etwa 140°C erhitzt.

9. Verfahren nach einem der Ansprüche 1-8, worin man pro Moläquivalent δ-Tocopherol bzw. der gesamten vorhandenen Nicht-α-Tocopherole etwa 2,5 bis etwa 10 Moläquivalente Mannich-Reagens verwendet.

10. Verfahren zur Regenerierung des überschüssigen Teils eines gemäss einem der Ansprüche 1 bis 9 im Ueberschuss verwendeten Mannich-Reagens, dessen Ueberschuss nach Beendigung des Aminomethylierungsverfahrens destillativ abgetrennt worden ist, dadurch gekennzeichnet, dass man dem destillativ abgetrennten überschüssigen Mannich-Reagens Wasser bei Raumtemperatur und unter Rühren zugibt, das resultierende wässrige Gemisch auf etwa 80°C erhitzt, und anschliessend Formaldehyd, vorzugsweise in Form von Paraformaldehyd, unter Rühren zugibt.

11. Verwendung eines gemäss Anspruch 10 regenerierten Mannich-Reagens in einem Verfahren zur Aminomethylierung von δ-Tocopherol oder von dieses enthaltenden Tocopherolgemischen.

## Claims

1. A process for the aminomethylation of δ-tocopherol or of tocopherol mixtures containing this, which process comprises using as a Mannich reagent one produced separately before the actual aminomethylation by reacting formaldehyde, especially paraformaldehyde, with a secondary amine in the complete or almost complete absence of a solvent.

2. A process according to claim 1, wherein between 0.7 and 1.2 mol equivalents of formaldehyde are used per mol equivalent of secondary amine.

3. A process according to claim 1 or 2, wherein the temperature of the reaction mixture consisting of formaldehyde and secondary amine is held in the range of about 70°C to about 90°C.

4. A process for the aminomethylation of δ-tocopherol or of tocopherol mixtures containing this, which process comprises using as a Mannich reagent one produced separately before the actual aminomethylation by reacting a diaminomethane produced from a secondary amine with formaldehyde, especially paraformaldehyde, as well as with water in about equimolar amounts.

5. A process according to any one of claims 1 to 4, wherein a di(C₁₋₆-alkyl)amine, a di(C₂₋₆-hydroxyalkyl)amine, a di(C₂₋₆-alkoxyalkyl)amine or a cyclic amine which is a 5- to 8-membered ring compound and which optionally contains a further hetero atom, especially oxygen or nitrogen, and/or can be substituted on the ring as well as on a further optionally present nitrogen atom, is used as the secondary amine.

6. A process according to claim 5, wherein the secondary amine is a di(C₁₋₆-alkyl)amine or a cyclic amine.

7. A process according to claim 6, wherein the secondary amine is 1-methyl-piperazine or morpholine.

8. A process according to any one of claims 1 - 7, wherein the reaction mixture consisting of the Mannich reagent and the δ-tocopherol or tocopherol mixture containing this is heated while stirring in a temperature range between about 100°C and about 140°C.

9. A process according to any one of claims 1 - 8, wherein about 2.5 to about 10 mol equivalents of Mannich reagent are used per mol equivalent of δ-tocopherol or of the total non-α-tocopherols present.

10. A process for the regeneration of the excess part of a Mannich reagent used in excess in accordance with any one of claims 1 to 9, which excess has been separated by distillation after completion of the aminomethylation, said process comprising adding water at room temperature and while stirring to the distillatively-separated excess Mannich reagent, heating the resulting aqueous mixture to about 80°C and subsequently adding formaldehyde, preferably in the form of paraformaldehyde, while stirring.

11. The use of a Mannich reagent regenerated according to claim 10 in a process for the aminomethylation of δ-tocopherol or of tocopherol mixtures containing this.

## Revendications

1. Procédé d'aminométhylation de δ-tocophérol ou de mélanges de tocophérols qui en contiennent, caractérisé en ce qu'on utilise comme réactif de Mannich un réactif qui a été préparé séparément avant l'aminométhylation proprement dite par réaction de formaldéhyde, en particulier de paraformaldéhyde, avec une amine secondaire, en l'absence complète ou presque complète de solvant.

2. Procédé selon la revendication 1, dans lequel on utilise par équivalent molaire de l'amine secondaire entre 0,7 et 1,2 équivalent molaire de formaldéhyde.

3. Procédé selon la revendication 1 ou 2, dans lequel on maintient la température du mélange réactionnel constitué de formaldéhyde et de l'amine secondaire dans un intervalle allant d'environ 70°C à environ 90°C.

4. Procédé d'aminométhylation de δ-tocophérol ou de mélanges de tocophérols qui en contiennent, caractérisé en ce qu'on utilise comme réactif de Mannich un réactif qui a été préparé séparément avant l'aminométhylation proprement dite par réaction d'un diaminométhane, préparé avec une amine secondaire, avec du formaldéhyde, en particulier du paraformaldéhyde, ainsi qu'avec de l'eau en quantités approximativement équimolaires.

5. Procédé selon l'une des revendications 1 à 4, dans lequel on utilise comme amine secondaire une di-alkyle en C₁ à C₆-amine, une di-hydroxyalkyle en C₁ à C₆-amine, une di-alcoxyalkyle en C₂ à C₆-amine ou une amine cyclique, qui est un composé cyclique à 5 à 8 chaînons et qui présente le cas échéant un autre hétéroatome, en particulier d'oxygène ou d'azote, et/ou peut être substitué sur son noyau, même sur un autre atome d'azote éventuellement présent.

6. Procédé selon la revendication 5, dans lequel l'amine secondaire est une di-alkyle en C₁ à C₆-amine ou une amine cyclique.

7. Procédé selon la revendication 6, dans lequel l'amine secondaire est la 1-méthyl-pipérazine ou la morpholine.

8. Procédé selon l'une des revendications 1 à 7, dans lequel on chauffe le mélange réactionnel constitué du réactif de Mannich et du δ-tocophérol ou du mélange de tocophérols qui en contient, tout en agitant, dans un intervalle de températures compris entre environ 100°C et environ 140°C.

9. Procédé selon l'une des revendications 1 à 8, dans lequel on utilise par équivalent molaire de δ-tocophérol ou selon les cas de l'ensemble des tocophérols non-α présents, d'environ 2,5 à environ 10 équivalents molaires de réactif de Mannich.

10. Procédé de régénération de la partie en excès d'un réactif de Mannich utilisée en excès selon l'une des revendications 1 à 9, dont on a séparé l'excès par distillation après la fin du procédé d'aminométhylation, caractérisé en ce qu'on ajoute au réactif de Mannich en excès séparé par distillation, de l'eau à la température ambiante et tout en agitant, en ce qu'on chauffe le mélange aqueux résultant à environ 80°C, puis en ce qu'on ajoute du formaldéhyde, de préférence sous forme de paraformaldéhyde, tout en agitant.

11. Utilisation d'un réactif de Mannich régénéré selon la revendication 10 dans un procédé d'aminométhylation de δ-tocophérol ou de mélanges de tocophérols qui en contiennent.
